(19) Europäisches Patentamt
European Patent Office
Office européen des brevets

(11) Publication number: **0 098 121**
**B1**

(12) **EUROPEAN PATENT SPECIFICATION**

(45) Date of publication of patent specification: **07.10.87**

(51) Int. Cl.$^4$: **C 07 F 15/00,** A 61 K 31/28

(21) Application number: **83303659.3**

(22) Date of filing: **24.06.83**

(54) **Platinum complexes, processes for their preparation, pharmaceutical compositions containing them and their use as medicaments.**

(30) Priority: **24.06.82 JP 108925/82**

(43) Date of publication of application:
**11.01.84 Bulletin 84/02**

(45) Publication of the grant of the patent:
**07.10.87 Bulletin 87/41**

(84) Designated Contracting States:
**DE FR GB**

(56) References cited:
**EP-A-0 001 126**
**EP-A-0 057 023**
**WO-A-82/00145**

**Patent Abstracts of Japan, vol.4, no.190, 26 Dezember 1980**

**The file contains technical information submitted after the application was filed and not included in this specification**

(73) Proprietor: **Kidani, Yoshinori**
**2-718, Mataho Kodan-jutaku 2-1, Mataho-cho**
**Nishi-ku Nagoya-shi Aichi-ken (JP)**

(72) Inventor: **Kidani, Yoshinori**
**2-718, Mataho Kodan-jutaku 2-1, Mataho-cho**
**Nishi-ku Nagoya-shi Aichi-ken (JP)**
Inventor: **Noji, Masahide**
**5-184, Fukazawa Kikko**
**Moriyama-ku Nagoya-shi Aichi-ken (JP)**

(74) Representative: **Watkins, Arnold Jack et al**
**European Patent Attorney Frank B. Dehn & Co.**
**Imperial House 15-19 Kingsway**
**London WC2B 6UZ (GB)**

**EP 0 098 121 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to platinum complexes, processes for their preparation, pharmaceutical compositions containing them and their use as medicaments. In particular, the invention relates to 1,2-diaminocyclohexaneplatinum (II) complexes of interest as medicaments.

It is known that certain platinum complexes are active against tumour cells [see for example, Nature, 222, 385 (1969); Platinum Rev., 15, No. 2, 42—51 (1971); ibid., 17, No. 1, 2—13 (1973); Chem. Biol. Interaction, vol. 5, 415—424 (1972); Bioinorg. Chem. vol. 2, 187—210 (1973); Res. Commun. Chem. Pathol. Pharmacol. vol. 7, 529—538; and US Patents 4,115,418; 4,196,846 and 4,200,583. In particular, cis dichloro-diammineplatinum (II) has been found to possess anti-tumour activity, but as a result of its toxic side effects, it has a rather low therapeutic index.

In EP—A—1,126 there are disclosed certain 1,2-diaminocyclohexaneplatinum (II) complexes having anti-tumour activity.

The present invention is based on the discovery of improved 1,2-diaminocyclohexaneplatinum (II) complexes which exhibit a good anti-tumour activity and low toxicity and are thus potentially of interest as medicaments.

Thus according to one feature of the present invention there is provided a platinum complex of the general formula:—

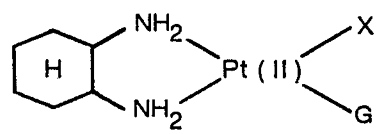

(I)

wherein X is a halogen atom and G is an anion derived from glucoronic acid (hereinafter referred to as D-gluc) of the formula:—

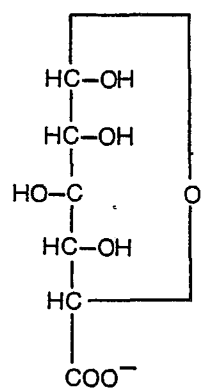

the configuration of the diaminocyclohexane being selected from cis-, trans-1- and trans-d-.

In formula I, X may represent, for example, a chlorine, bromine, iodine or fluorine atom and G is the D-glucuronic acid anion of the above-identified formula referred to as "D-gluc". X preferably represents chlorine, but conveniently represents bromine or iodine.

By way of example, therefore, the complexes of formula (I) include Pt(II)Cl(D-gluc) (dach), Pt(II)Br(D-gluc) (dach), Pt(II)I(D-gluc) (dach) and Pt(II)F(D-gluc) (dach).

In this specification, diaminocyclohexane is referred to as "dach" and its configuration is selected from cis-, trans-1- and trans-d. Each of said configurations may be employed in relation to each individual definition of X.

Complexes of the present invention which have been tested, particularly complexes of formula I in which X represents a chlorine atom such as Pt(II)Cl(D-gluc) (trans-1-dach) [i.e. a complex of formula I in which X represents a chlorine atom and the diaminocyclohexane moiety is of the trans-1-configuration], exhibit at least moderate water-solubility and good therapeutic activity when compared with known platinum complexes having anti-tumour activity.

According to a further feature of the present invention there is provided a process for the preparation of a platinum complex as hereinbefore defined characterized in that a compound of the formula:—

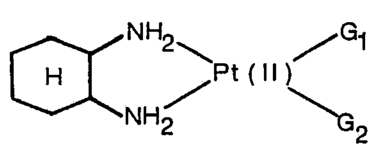

(II)

(wherein one of $G_1$ and $G_2$ is as hereinbefore defined for G and the other of $G_1$ and $G_2$ represents a leaving ligand) is reacted with a compound of the formula:—

$$MX_n \qquad (III)$$

(wherein X is as hereinbefore defined, M represents a stabilising metal cation and n represents the charge on the cation) whereby a platinum complex of formula I as hereinbefore defined is obtained.

A compound of formula II is preferably used in which $G_1$ and $G_2$ are each as hereinbefore defined for G. A compound of formula III is preferably used in which M represents an alkaline earth metal or an alkali metal, especially an alkali metal, for example sodium or more especially potassium. Where M represents an alkali metal n is 1. The compound of formula III is preferably water soluble.

The complexes of formula (I) are thus preferably prepared by the reaction of Pt(II)(D-gluc)$_2$(dach) with KX (wherein X is as hereinbefore defined). The reaction may conveniently be effected in water, for example, about ambient temperature, but especially at a temperature of from 0 to 15°C for 10—20 hours. After completion of the reaction, the desired product may be recovered from the reaction solution, by known techniques, for example, by column chromatography using for example Amberlite R-120, Amberlite IRA-400 (commercial products of Rohm and Haas Co., U.S.A.), or Sephadex G-10 (commercial product of Pharmacia Fine Chemicals AB, Sweden).

The starting materials for use in preparing the complexes of the present invention may be prepared by methods analogous to those well known in the art. Thus for example Pt(II)(D-gluc)$_2$(dach) used as starting material for the preparation of the complexes of formula (I) may be obtained in conventional manner [for example, as described in US Patent 4,200,583].

According to a further feature of the present invention there is provided a pharmaceutical composition comprising as active ingredient at least one platinum complex as hereinbefore defined in association with a pharmaceutical carrier or excipient.

The compositions may be presented in a form suitable for oral, rectal or parenteral administration. Thus, for example, compositions for oral administration may be solid or liquid and may take the form of granules, tablets, coated tablets, capsules, syrups, emulsions, suspensions or drops, such compositions comprising carriers or excipients conventionally used in the pharmaceutical art. Thus, for example, suitable tabletting excipients include lactose, potato and soluble starches and magnesium stearate.

For parenteral administration, the carrier may be a sterile, parenterally acceptable liquid such as sterile water, or a parenterally acceptable oil e.g. arachis oil, contained in ampoules. Compositions for rectal administration may take the form of suppositories, the carrier comprising a suppository base.

Advantageously, the compositions may be formulated as dosage units, each unit being adapted to supply a fixed dose of active ingredient. Tablets, coated tablets, capsules, suppositories and ampoules are examples of suitable dosage unit forms.

The present invention also provides platinum complexes of formula I as hereinbefore defined for use as an anti-tumour agent.

With reference to the accompanying drawings Figs. 1 and 2 show the infrared and ultraviolet absorption spectra respectively of Pt(II)Cl(D-gluc) (trans-1-dach). Figs. 3—7 show the HPLC charts obtained for Pt(II)Cl(D-gluc) (trans-1-dach), Pt(II)Cl(D-gluc) (cis-dach), Pt(II)Cl(D-gluc) (trans-d-dach), Pt(II)Br(D-gluc) (trans-1-dach) and Pt(II)I(D-gluc) (trans-1-dach) respectively.

The following non-limiting Examples illustrate the invention. "Amberlite" and "Sephadex" are registered Trade Marks.

## Example 1

Pt(II)(D-gluc)$_2$(trans-1-dach) [150 mg; $2.156 \times 10^{-4}$ mol] was dissolved in water (5 ml) and potassium chloride (16.1 mg, dissolved in 1 ml of water) was added, the reaction being effected at 5—7°C for about 16 hours. The reaction solution was filtered and the filtrate was passed through a column (1 × 15 cm) packed with Amberlite R-120 (0.5 g) and another column (1 × 15 cm) packed with Amberlite IRA-400 (0.5 g) to remove potassium chloride, followed by column chromatography using a column (2.6 × 40 cm) packed with Sephadex G-10 (40 g) eluted with water. The resultant effluent was divided into 100 fractions (each 3.3 ml). The ultraviolet absorption at 290 nm was measured and Fraction Nos. 30—39 corresponding to the peak which eluted after the starting material were collected and combined. The combined fractions were concentrated and freeze-dried to obtain Pt(II)Cl(D-gluc) (trans-1-dach) having the following physical properties in a yield of about 20%:

| Elemental analysis: | H | C | N |
|---|---|---|---|
| Calculated | 4.54 | 25.92 | 5.04 |
| Found | 4.50 | 26.88 | 4.76 |

The ultraviolet and infrared absorption spectra of the product are shown respectively in Figs. 2 and 1. A HPLC chart shown in Fig. 3 was obtained by treating the product under the following conditions:

| | | |
|---|---|---|
| Eluting solution | : | 0.1M $Na_2SO_4$ (pH=6.02) |
| Flow rate | : | 1.0 ml/min |
| Chart speed | : | 2.5 mm/min. |

The desired product was found by the maximum peak observed after the stay of 26.5—26.6 minutes. The thus-obtained product exhibited the following anti-tumour activity.

CDF mice (each group consisting of 6 mice) were used as test animals. On each occasion, $10^5$ cells of L 1210 were administered into the abdominal cavity of the animal. On the same day and on the 5th and 9th days after this, samples of the test compound were administered to the animals to investigate the extension in the number of survival days over control groups of mice i.e. T/C % shown in the following table:

| Dose (mg/kg)/day | T/C % |
|---|---|
| 50 | 86 |
| 25 | 285 (4/6) |
| 12.5 | 301 (3/6) |
| 6.25 | 249 (2/6) |
| 3.12 | 150 |

Example 2

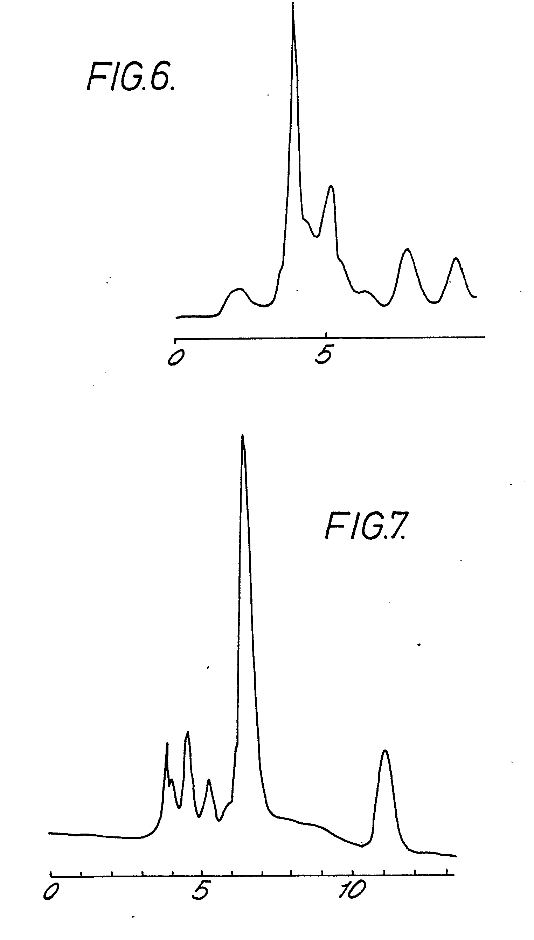

Analogous treatments to those described in Example 1 were repeated, using respectively Pt(II)(D-gluc)$_2$(cis-dach) and Pt(II)(D-gluc)$_2$(trans-d-dach) as starting compounds, to obtain the HPLC charts shown respectively in Figs. 4 and 5. The reaction solutions were filtered to obtain the filtrates which were chromatographed by HPLC. The maximum peak shown in Fig. 4 and the second peak shown in Fig. 5 (from the right hand side) show respectively Pt(II)Cl(D-gluc) (cis-dach) and Pt(II)Cl(D-gluc) (trans-d-dach).

Example 3

Similar procedures to those described in Example 1 were repeated by using respectively potassium bromide and potassium iodide instead of potassium chloride. On each occasion, the reaction solution was filtered and the filtrate was chromatographed by HPLC the charts obtained being shown in Figs. 6 and 7 respectively. The second peak from the right-hand side in Fig. 6 and the maximum peak in Fig. 7 show respectively Pt(II)Br(D-gluc) (trans-1-dach) having the following elemental analysis:—

Calculated: H, 3.95%, C, 24.74% and N, 4.81%
Found: H, 3.90%, C, 25.02% and N, 4.67%

and Pt(II)I(D-gluc) (trans-l-dach) having the following elemental analysis:—

Calculated: H, 4.10%, C, 21.66% and N, 4.21%
Found: H, 3.90%, C, 21.67% and N, 4.12%

**Claims**

1. A platinum complex of the general formula:—

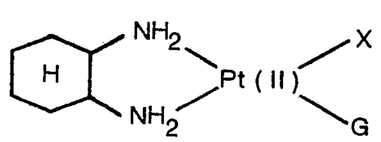

(I)

wherein X is a halogen atom and G is an anion derived from glucaronic acid of the formula:—

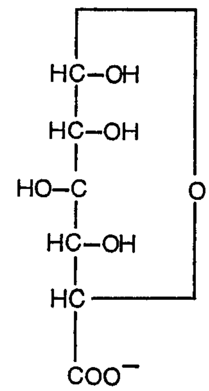

the configuration of the diaminocyclohexane being selected from cis-, trans-l- and trans-d- forms.

2. A complex as claimed in claim 1 wherein X represents a chlorine atom.

3. A complex as claimed in claim 1 wherein X represents a bromine or iodine atom.

4. A complex as claimed in claim 1 wherein X represents a chlorine atom and the diaminocyclohexane moiety is of the trans-L-configuration.

5. A process for the preparation of a platinum complex as defined in claim 1 characterized in that a compound of the formula:—

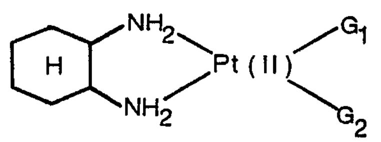

(II)

(wherein one of $G_1$ and $G_2$ is as defined for G in claim 1 and the other of $G_1$ and $G_2$ represents a leaving ligand) is reacted with a compound of the formula:—

$$MX_n \qquad (III)$$

(wherein X is as defined in claim 1, M represents a stabilising metal cation and n represents the charge on the cation).

6. A process as claimed in claim 5 wherein a compound of formula II is used in which $G_1$ and $G_2$ are each as defined for G in claim 1.

7. A process as claimed in claim 5 or claim 6 wherein a compound of formula III is used in which M represents an alkali metal and n is 1.

8. A process as claimed in claim 7 wherein the alkali metal is potassium.

9. A pharmaceutical composition comprising as active ingredient at least one platinum complex as defined in claim 1 in association with a pharmaceutical carrier or excipient.

10. A platinum complex as defined in claim 1 for use as an anti-tumour agent.

**Patentansprüche**

1. Platinkomplex der allgemeinen Formel:

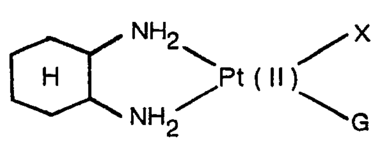

(I)

worin X ein Halogenatom bedeutet und G ein Anion der Glucoronsäure der Formel:

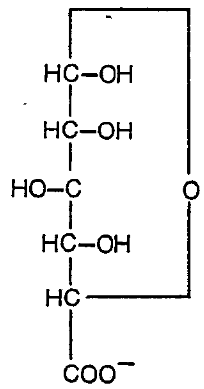

darstellt, wobei das Diaminocyclohexan cis-, trans-l- oder trans-d-Konfiguration besitzt.

2. Komplex nach Anspruch 1, worin X ein Chloratom bedeutet.

3. Komplex nach Anspruch 1, worin X ein Brom- oder Jodatom bedeutet.

4. Komplex nach Anspruch 1, worin X ein Chloratom bedeutet und die Diamonocyclohexaneinheit in der trans-L-Konfiguration vorliegt.

5. Verfahren zur Herstellung eines Platinkomplexes nach Anspruch 1, dadurch gekennzeichnet, daß man eine Verbindung der Formel

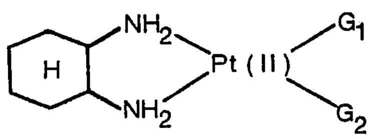

(II)

(worin einer der Reste $G_1$ oder $G_2$ die in Anspruch 1 für G angegebene Bedeutung besitzt, und der andere der Reste ein austretender Ligand ist) mit einer Verbindung der Formel:

$$MX_n \qquad \text{(III)}$$

(worin X wie in Anspruch 1 definiert ist, M ein stabilisierendes Metallkation und n die Ladung des Kations bedeuten), umsetzt.

6. Verfahren nach Anspruch 5, worin man eine Verbindung der Formel II einsetzt, in der $G_1$ und $G_2$ jeweils die für G in Anspuch 1 angegebene Bedeutung besitzen.

7. Verfahren nach Anspruch 5 oder 6, worin man eine Verbindung der Formel III einsetzt, in der M ein Alkalimetall bedeutet und n für 1 steht.

8. Verfahren nach Anspruch 7, worin das Alkalimetall Kalium ist.

9. Pharmaceutisches Mittel, das als aktiven Bestandteil mindestens einen Platinkomplex nach Anspruch 1 in Verbindung mit einem pharmazeutischen Träger oder Exzipienten enthält.

10. Platinkomplex nach Anspruch 1 zur Verwendung als Antitumoragens.

**Revendications**

1. Complexe de platine de formule générale:

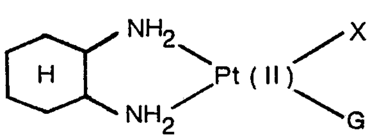

(I)

dans laquelle X est un atome d'halogène et G est un anion dérivé de l'acide glucoronique de formule:

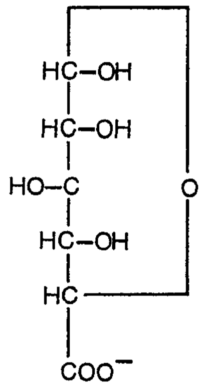

la configuration du diaminocyclohexane étant choisie parmi les formes cis, trans-l et trans-d.

2. Complexe suivant la revendication 1, dans lequel X représenté un atome de chlore.

3. Complexe suivant la revendication 1, dans lequel X représenté un atome de brome ou d'iode.

4. Complexe suivant la revendication 1, dans lequel X représenté un atome de chlore et le groupement diaminocyclohexane a la configuration trans-L.

5. Procédé de préparation d'un complexe de platine tel que défini dans la revendication 1, caractérisé en ce qu'on fait réagir un composé de formule:

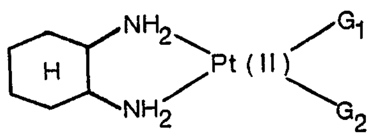

(II)

(dans laquelle l'un de $G_1$ et $G_2$ a la définition donnée pour G dans la revendication 1 et l'autre représente un ligand partant) avec un composé de formule:

$$MX_n \quad (III)$$

(dans laquelle X est tel que défini dans la revendication 1, M représente un cation de métal stabilisant et *n* représente la charge portée par le cation).

6. Procédé suivant la revendication 5, dans lequel on utilise un composé de formule II dans lquelle $G_1$ et $G_2$ ont chacun la définition donnée pour G dans la revendication 1.

7. Procédé suivant la revendication 5 ou la revendication 6, dans lequel on utilise un composé de formule III où M représente un métal alcalin et *n* est égal à 1.

8. Procédé suivant la revendication 7, dans lequel le métal alcalin est le potassium.

9. Composition pharmaceutique comprenant comme ingrédient actif au moins un complexe de platine tel que défini dans la revendication 1 en association avec un support ou excipient pharmaceutique.

10. Complexe de platine suivant la revendication 1, destiné à être utilisé comme agent anti-tumoral.

FIG.1.

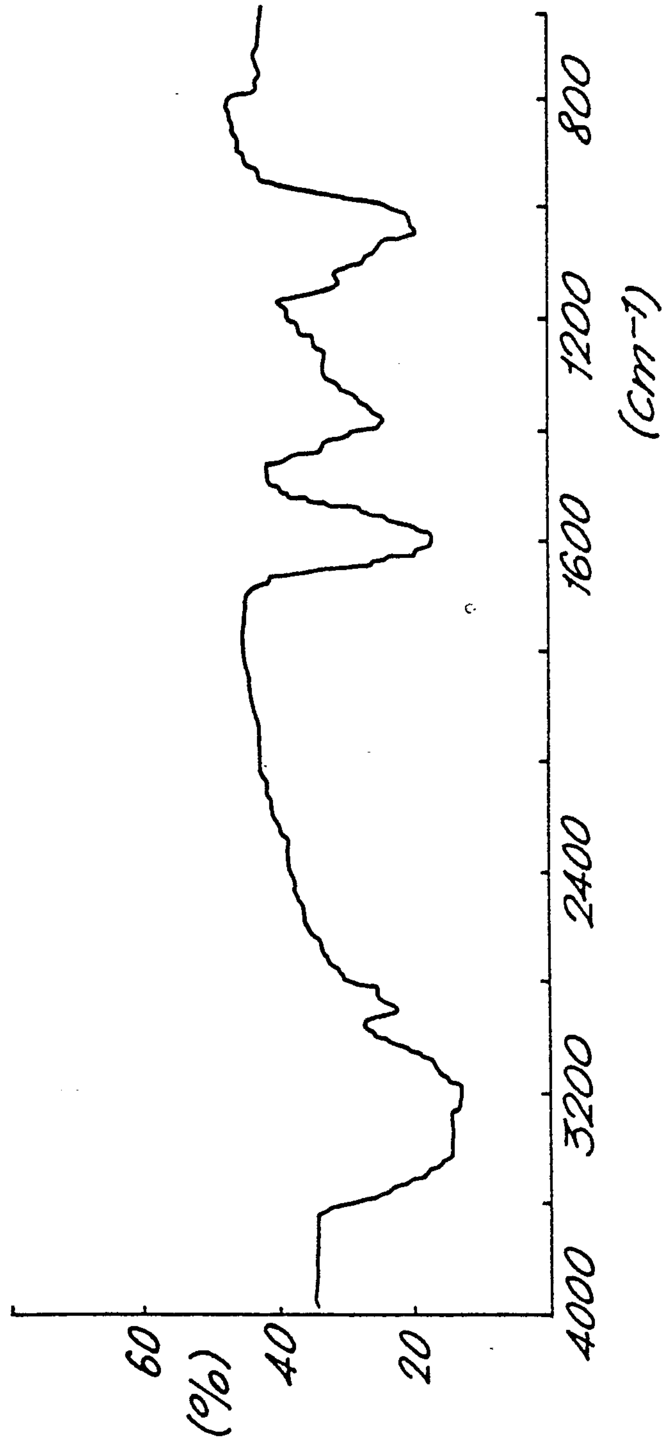

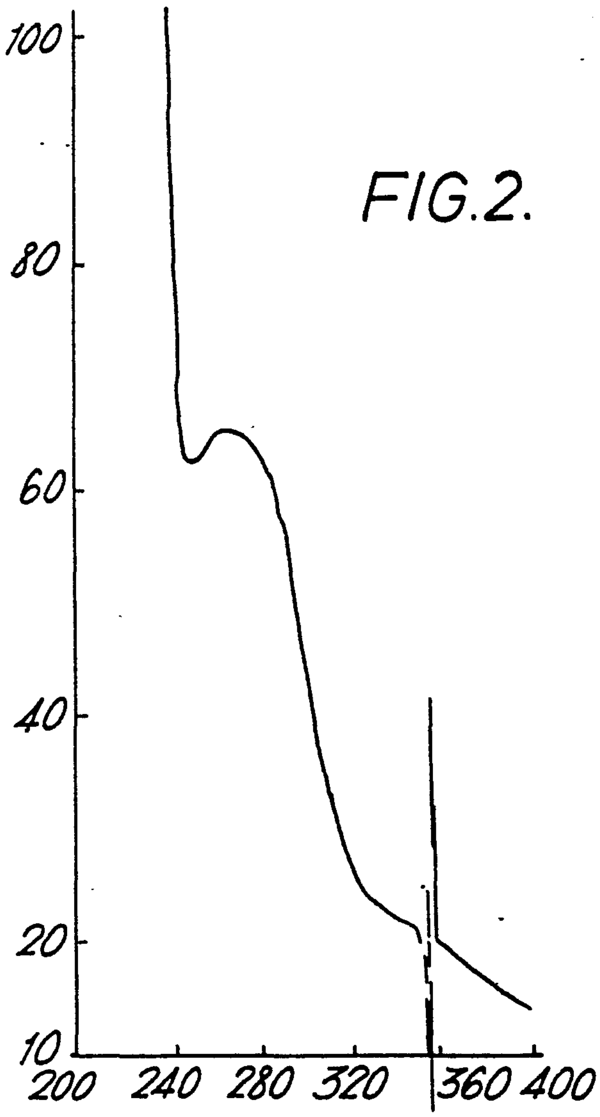


FIG.2.

FIG.3.

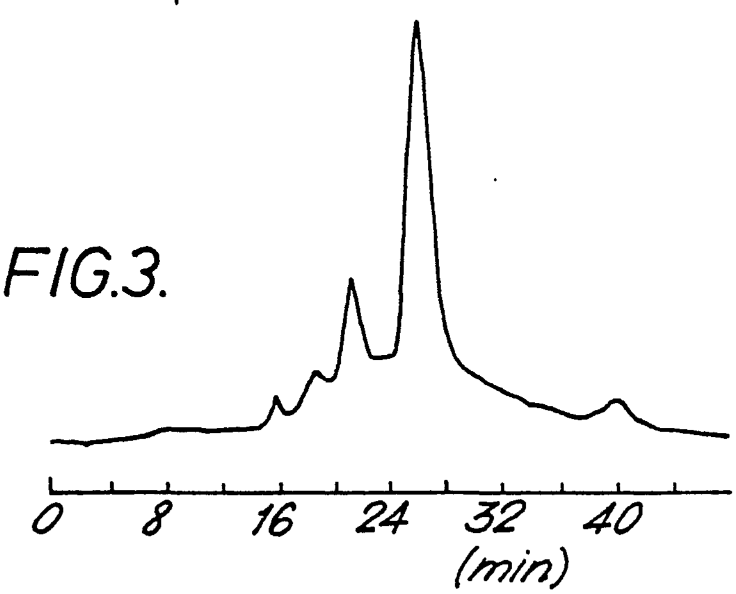

FIG.4.

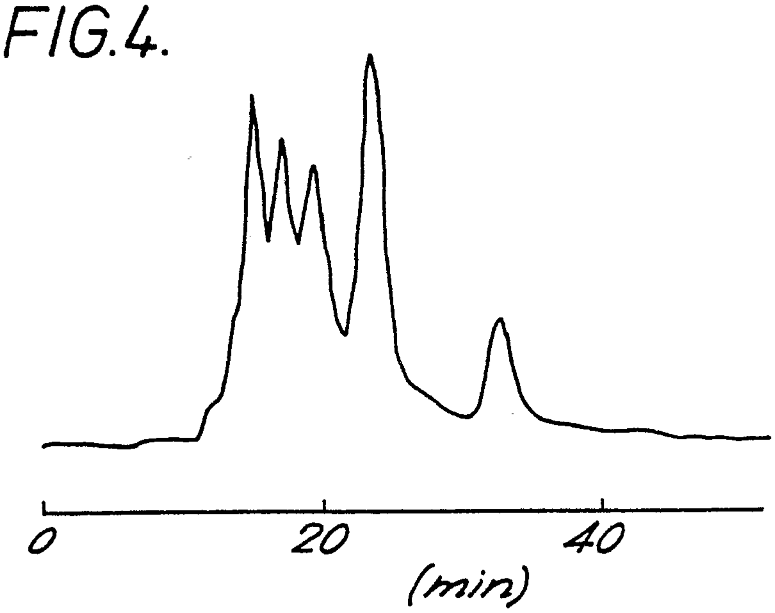


FIG.5.

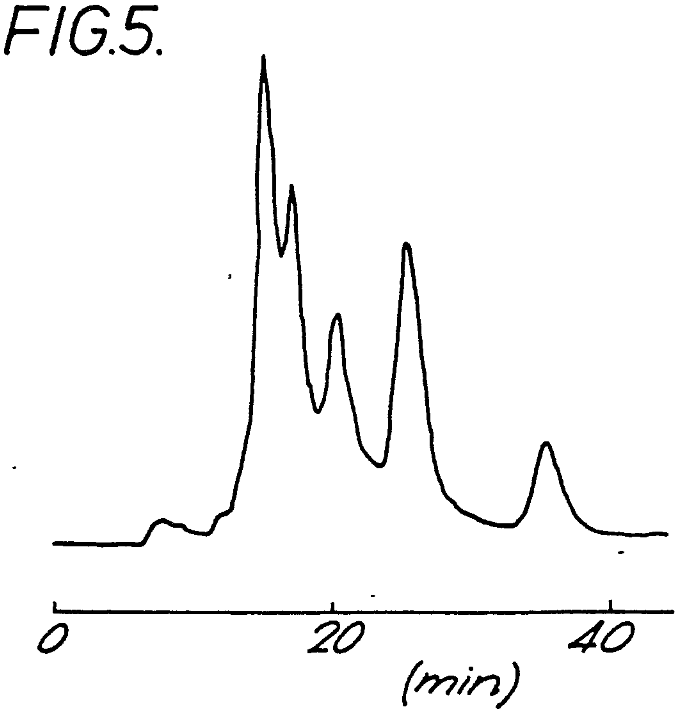

FIG.6.

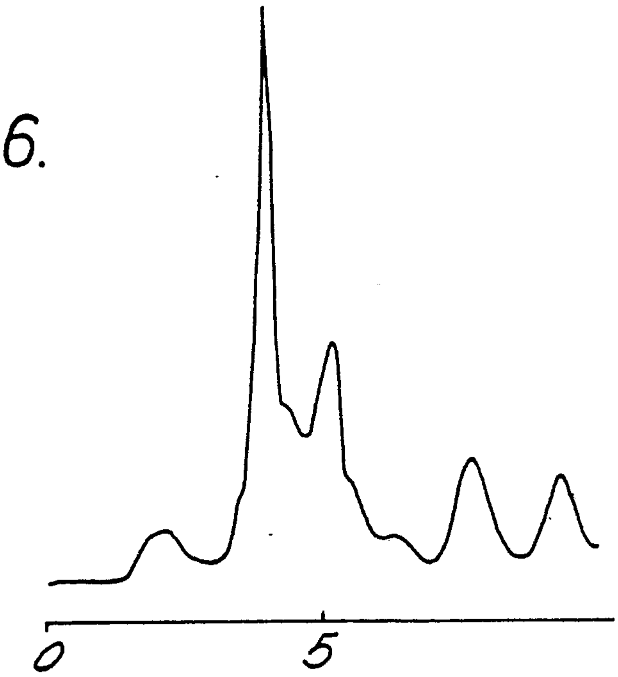


FIG.7.